# EUROPÄISCHE PATENTSCHRIFT

(11) **EP 0 170 998 B1**
(45) Veröffentlichungstag und Bekanntmachung des Hinweises auf die Patenterteilung: **07.04.1999**
(21) Anmeldenummer: 85109394.8
(22) Anmeldetag: 26.07.1985
(51) Int. Cl.: G01N 27/26

(54) **Verfahren zur elektrochemischen Bestimmung der Sauerstoffkonzentration**
Method for the electrochemical determination of oxygen concentration
Procédé pour la détection électrochimique de la concentration en oxygène

(30) Priorität: 10.08.1984 DE 3429583
(43) Veröffentlichungstag der Anmeldung: 12.02.1986
(73) Patentinhaber: Pacesetter AB, 175 84 Järfälla (SE)
(72) Erfinder: Mund, Konrad, Dr., D-8525 Uttenreuth (DE); Preidel, Walter, Dr., D-8520 Erlangen (DE); Rao, J. Raghavendra, Dr., D-8520 Erlangen (DE); Richter, Gerhard, Dr., D-8520 Erlangen (DE)
(74) Vertreter: Lettström, Richard Wilhelm

(56) Entgegenhaltungen:
- EP-A- 0 071 152
- WO-A-83/01687
- DE-A- 2 156 669
- FR-A- 2 345 170
- GB-A- 2 019 580
- GB-A- 2 059 597
- US-A- 4 166 775

## Beschreibung

Die Erfindung betrifft ein Verfahren zur elektrochemischen Bestimmung der Saurestoffkonzentration, insbesondere in biologischem Material, mittels eines Sauerstoffsensors mit einer Meßelektrode und einer Gegenelektrode.

In der modernen Medizin werden für die Therapie von Herzrhytmusstörungen in zunehmendem Maße implantierbare Herzschrittmacher eingesetzt. Dabei wird der Herzmuskel durch kurze Stromimpulse stimuliert, die mittelse einer Reizelektrode übertragen werden. Die implantierten Herzschrittmacher arbeiten im allgemeinen mit konstanter Frequenz und simulieren somit die Steuerung des gesunden Herzens, das seine Schlagzahl bei Bedarf ändert, nur unvolkommen. Deshalb wird eine physiologische Steurung der Herzschrittmacher angestrebt, wozu beispielsweise die Sauerstoffkonzentration im Blut oder Gewebe als Steuerparameter dienen kann. Dabei wird dann die Frequenz der Reizimpulse der Sauerstoffkonzentration bzw. dem O₂-Partialdruck angepaßt, um das Herz bei mangelhafter O₂-Versorgung schneller schlagen zu lassen. Zu diesem Zweck muß also die O₂-Konzentration kontinuierlich gemessen werden, d.h. es wird ein implantierbarer Sauerstoffsensor benötigt. Neben der Verwendung in Herzschrittmachern eignen sich Sauerstoffsensoren auch zur Bestimmung des Sauerstoffgehaltes bei der Respiration und können somit bei der Anästhesie und der intensiven Patientenüberwachung eingesetzt werden.

Bislang wird die Sauerstoffkonzentration im Blut bzw. Gewebe im allgemeinen extrakorporal gemessen, in vivo dagegen nur in sehr begrenztem Umfang und nur kurzzeitig. Als Grundlage dient dabei im westenlichen das elektrochemische Prinzip, wozu eine sogenannte Clark-Zelle verwendet wird (siehe US-Patentschriften 2 913 386, 3 260 656 und 4 076 596). Bei einem derartigen Sauerstoffsensor ist vor der Meßelektrode eine Membran angeordnet, wodurch sich an der Elektrode ein Diffusionsgrenzstrom einstellt, d.h. es wird ein konzentrationsproportionales Meßsignal erhalten.

Für eine langfristige Implantation ist die vorstehend genannte Meßmethodik nicht geeignet, da der Grenzstrom stark von der Diffusionsschicht abhängt und die sich im Körper nach der Implantation zwangsläufig bildende Bindegewebsschicht somit das Meßsignal verfälschen kann. Die prinzipielle Lösung dieses Problems besteht darin, langfristig implantierbare Membranen zu verwenden. Für diesen Zweck geeignete Membranen sind bislang aber noch nicht bekannt.

Aufgabe der Erfindung ist es, ein Verfahren anzugeben, das - unter Verwendung eines Sauerstoffsensors, mit einer Meß- und einer Gegenelektrode - auch bei einer Implantation des Sensors eine Bestimmung der Sauerstoffkonzentration über lange Zeiträume hinweg ermöglicht.

Eine weitere Aufgabe der Erfindung ist es, eine Anordnung zur Durchführung des Verfahrens anzugeben.

Dies wird erfindungsgemäß durch ein Verfahren zur elektrochemischen Bestimmung der Sauerstoffkonzentration in Gewebe oder Blut, ohne die Verwendung einer Membran, mittels eines Sauerstoffsensors mit einer Meßelektrode bestehend aus einer Gold-, Glaskohlenstoff- oder Pyrographitelektrode und mit einer Gegenelektrode aus einem Material, das sowohl inert als auch biokompatibel ist und eine hohe Doppelschichtkapazität gegenüber der Meßelektrode aufweist erreicht, wobei der Meßelektrode zyklisch zwei Potentiale aufgeprägt werden, wobei das eine Potential, das Meßpotential, im Bereich - 1,4 V ≤ ϕ_{Ag/AgCl} ≤ -0,4 V und das andere Potential, das Erholungspotential, im Bereich -0,2V ≤ ϕ_{Ag/AgCl} ≤ +0,2 V liegt, die Verweildauer beim Meßpotential zwischen 5 und 100 ms beträgt und klein ist im Vergleich zur Zyklusdauer und der während der Meßperiode fließende Strom als Meßsignal ausgewertet wird.

Die erfindungsgemäße Anordnung umfaßt
a) ein implantierbaren Sauerstoffsensor mit einer Meßelektrode bestehend aus einer Gold-, Glaskohlenstoff- oder Pyrographitelektrode und mit einer Gegenelektrode aus einem Material, das sowohl inert als auch biokompatibel ist und eine hohe Doppelschichtkapazität gegenüber der Meßelektrode aufweist,
b) Mittel zur zyklischen aufprägeln der Meßelektrode von zwei Potentiale, wobei das eine Potential, das Meßpotential, im Bereich -1,4 V ≤ ϕ_{Ag/AgCl} ≤ -0,4 V und das andere Potential, das Erholungspotential, im Bereich -0,2 V ≤ ϕ_{Ag/AgCl} ≤ +0,2 V liegt; und
c) Mittel zur Auswertung des während der Meßperiode fließende Stroms als Meßsignal,
wobei die Verweildauer beim Meßpotential zwischen 5 und 100 ms beträgt und klein ist im Vergleich zur Zyklusdauer.

Das erfindungsgemäße Verfahren verwendet keine Membran und vermeidet somit die damit verbundenen Nachteile. Das Grundprinzip dieses Verfahrens besteht darin, daß - bei der Messung - der unmittelbaren Umgebung der Meßelektrode, beispielsweise dem Gewebe, so wenig Sauerstoff wie möglich entnommen wird, um diese Umgebung nicht wesentlich an Sauerstoff abzureichern. Auf diese Weise degeneriert das Gewebe nicht, d.h. es wirt nicht in Bindegewebe ungewandelt, sondern bleibt intakt. Ein entsprechender Sauerstoffsensor eignet sich somit insbesondere für eine langfristige Implantation, d.h. für einen Zeitraum von mehreren Jahren.

Praktisch wird das dem erfindungsgemäßen Verfahren zugrundeliegende Prinzip in der Weise verwirklicht, daß der Meß- bzw. Sensorelektrode zunächst - für relativ lange Zeit - ein Potential aufgeprägt wird, bei dem keine Sauerstoffumsetzung und keine Elektrodenkorrosion erfolgt, nämlich das sogenannte Erholungspotential. Dann wird der Elektrode ein Meßpotential in der Weise aufgeprägt, daß die Sauerstoffumsetzung in einem Maße erfolgt, wie es für die Messung erforderlich ist, der O₂-Verbrauch dabei aber möglichst gering bleibt. Dies wird durch eine relativ kurze Verweildauer beim Meßpotential erreicht, d.h. durch eine Dauer des Meßpotentials, die klein ist im Vergleich mit der Zyklusdauer bzw. mit der Erholungsphase. Die Erholungsphase ist deshalb relativ lang, damit der während der Messung erfolgende Sauerstoffverlust durch Nachlieferung aus dem Gewebe ausgeglichen wird.

WO 83/01687 beschreibt ein Verfahren zur elektrochemischen Bestimmung der Sauerstoffkonzentration in stillem Wasser mittels eines Sensors mit einer Meßelektrode, einer Gegenelektrode und einer Membran. Der Sensor wird zyklisch ein Sauerstoffumsetzungspotential (On-Periode) und ein Erholungspotential (Off-Periode) aufgeprägt. Als Meßsignal wird der während der "On-Periode" und während wenigstens einen Teil der "Off-Periode" fließende Strom ausgewertet. In einem Beispiel werden eine "On-Periode" von 1 s und ein Teil einer "Off-Periode" von 1 min integriert. Der Sensor wird nicht in biologischem Material verwendet.

Die Zyklusdauer beträgt beim erfindungsgemäßen Verfahren vorteilhaft zwischen 0,5 und 10 s und vorzugsweise zwischen 1 und 5 s. Die Verweildauer beim Meßpotential liegt zwischen 5 und 100 ms und vorzugsweise zwischen 10 und 50 ms. Die Verweildauer am Meßpotential ist vorzugsweise wenigstens um den Faktor 20 kleiner als die Zyklusdauer.

Das Meßpotential liegt beim erfindungsgemäßen Verfahren im Bereich zwischen -0,4 und -1,4 V, bezogen auf das Potential der Ag/AgCl-Elektrode (bzw. zwischen +0,2 und -0,8 V, bezogen auf das Potential der reversiblen Wasserstoffelektrode); das Erholungspotential liegt im Bereich zwischen +0,2 und -0,2 V, bezogen auf das Potential der Ag/AgCl-Elektrode (bzw. zwischen 0,8 und 0,4 V, bezogen auf das Potential der reversiblen Wasserstoffelektrode). Durch diese Potentialbereiche ist gewährleistet, daß physiologische Begleitsubstanzen, wie Glucose, Harnstoff und Aminosäuren, die Sauerstoffmessung im Körper nicht beeinträchtigen.

Beim erfindungsgemäßen Verfahren, das ohne Störung und ohne Beeinträchtigung der Elektrodenumgebung verläuft, wird der von der Sauerstoffkonzentration abhängige Reduktionsstrom erfaßt und als Meßsignal ausgewertet. Vorteilhaft wird dabei der während der Meßperiode fließende Strom integriert und das Ladungsintegral als Meßsignal ausgewertet.

Die Auswertung des Stromes bzw. seine Integration erfolgt vorteilhaft etwas zeitverzögert, d.h. mit einer zeitlichen Verzögerung in bezug auf den Beginn der Meßperiode. Auf diese Weise wird bei der Auswertung die Umladung der Elektrodendoppelschichtkapazität, die in den ersten 2 bis 5 ms erfolgt, eliminiert, d.h. es wird nur der eigentliche Reaktionsstrom berücksichtigt. Vorteilhaft erfolgt die Auswertung des Stromes 2 bis 40 ms, vorzugsweise 10 bis 15 ms, nach Beginn der Meßperiode.

Die Meßelektrode des beim erfindungsgemäßen Verfahren eingesetzten Sauerstoffsensors ist vorzugsweise eine (glatte) Glaskohlenstoffelektrode. Glaskohlenstoff, der sich bereits als Material für die Reizelektrode von Herzschrittmachern bewährt hat (siehe beispielsweise DE-OS 26 13 072), ist körperverträglich und wird im Körper von Bindegewebe mit einer Dicke von weniger als 100 µm umhüllt. Durch eine derartige Bindegewebsschicht wird die Diffusion von Sauerstoff zur Oberfläche der Meßelektrode nicht behindert. Da Glaskohlenstoff katalytisch wenig aktiv ist, werden darüber hinaus körpereigene Substanzen kaum adsorbiert und lösen somit keine Reaktionen aus. Neben Glaskohlenstoff können als Material für die Meß- bzw. Sensorelektrode auch Gold und Pyrographit verwendet werden. Elektrodenmaterialien der genannten Art sind im übrigen an sich bekannt (vgl. dazu: F. Kreuzer, H.P. Kimmich und M. B 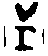 ezina in J. Koryta "Medical and Biological Applications of Electrochemical Devices", John Wiley & Sons Ltd., 1980, Seite 173 ff.).

Die Gegenelektrode besteht aus einem Material, das sowohl inert als auch biokompatibel ist und eine hohe Doppelschichtkapazität gegenüber der Meßelektrode aufweist. Vorzugsweise ist die Gegenelektrode eine Platinelektrode. Als weitere Elektrodenmaterialien können beispielsweise aktivierter Glaskohlenstoff, d.h. Glaskohlenstoff mit einer mikroporösen Oberflächenschicht (siehe dazu DE-OS 26 13 072), und Titannitrid (DE-OS 33 00 668) verwendet werden.

Neben der Meß- und der Gegenelektrode kann der beim erfindungsgemäßen Verfahren eingesetzte Sauerstoffsensor vorteilhaft auch noch eine Bezugselektrode aufweisen. Als Bezugselektrode dient dabei vorzugsweise eine Ag/AgCl-Elektrode. Eine derartige Elektrode kann beispielsweise durch Anodisieren eines Silberdrahtes in einer chloridhaltigen Lösung oder durch Eintauchen eines Silber drahtes in eine Silberchloridschmelze hergestellt werden.

Der Sauerstoffsensor selbst besteht dann aus einer Zwei- oder Dreielektrodenanordnung, wobei die Elektroden vorteilhafterweise in Form einer kleinen Kathetersonde zusammengebaut sind. Auf diese Weise kann die Elektrodenanordnung leicht ins Blut, beispielsweise durch Venen, oder ins Gewebe eingebracht werden.

Das erfindungsgemäße Verfahren kann sowohl bei Sauerstoffsensoren zur Steuerung von Herzschrittmachern als auch bei anderen Anwendungen in der Medizin verwendet werden, insbesondere bei der Anästhesie und der Patientenüberwachung.

Anhand von Ausführungsbeispielen und Figuren soll die Erfindung noch näher erläutert werden.

Eine bevorzugte Ausführungsform des beim erfindungsgemäßen Verfahren eingesetzten Sauerstoffsensors weist folgende Elektroden auf: Eine Meßelektrode aus glattem, d.h. nicht aktiviertem Glaskohlenstoff, ein Platinblech als Gegenelektrode und eine Ag/AgCl-Bezugselektrode (Elektrolyt: 3 M KCl). Diese Elektroden sind bei Versuchsmustern des Sauerstoffsensors in einer Zelle aus Glas angeordnet. Bei den Versuchen wurde zum Teil mit einer rotierenden Meßelektrode (200 U/min) in Form eines zylinderförmigen Stabes gearbeitet (Durchmesser: 2 mm; Mantelfläche: 0,19 cm²). Bei scheibenförmigen Meßelektroden, welche ebenfalls einen Durchmesser von 2 mm aufweisen, beträgt die Fläche 0,03 cm².

Bei einer Dreielektrodenanordnung der vorstehend genannten Art kann die Meßelektrode auch aus Gold bestehen, d.h. es wird ein Goldstab verwendet. Bei einem Sauerstoffsensor mit einer Zweielektrodenanordnung wird beispielsweise eine glatte Glaskohlenstoffelektrode als Meßelektrode mit einer großflächigen Scheibe aus aktiviertem Glaskohlenstoff (Durchmesser: 4 cm) als Gegenelektrode kombiniert.

Mit Sauerstoffsensoren der vorstehend genannten Art werden deutlich reproduzierbare und von der Sauerstoffkonzentration abhängige Meßsignale erhalten und somit auch gute Eichkurven, und zwar auch bei der Verwendung verschiedener Elektrolyte. Als Elektrolyt diente beispielsweise eine physiologische Kochsalzlösung (mit 0,9 % NaCl und 0,1 % NaHCO₃); etwa 150 ml dieser Lösung wurden jeweils eingesetzt (Grundelektrolyt). Der Grundelektrolyt kann auch physiologische Substanzen enthalten, wie Glucose, Harnstoff und Aminosäuren. Zur Herstellung eines derartigen Elektrolyten werden dem Grundelektrolyt 1 g/l Glucose, 388 mg/l Harnstoff und 398,3 mg/l eines Aminosäuregemisches (aus 16 Aminosäuren, jeweils in der physiologisch maximalen Konzentration) zugesetzt. Als Elektrolyt kann ferner auch Rinderserum oder Humanserum dienen.

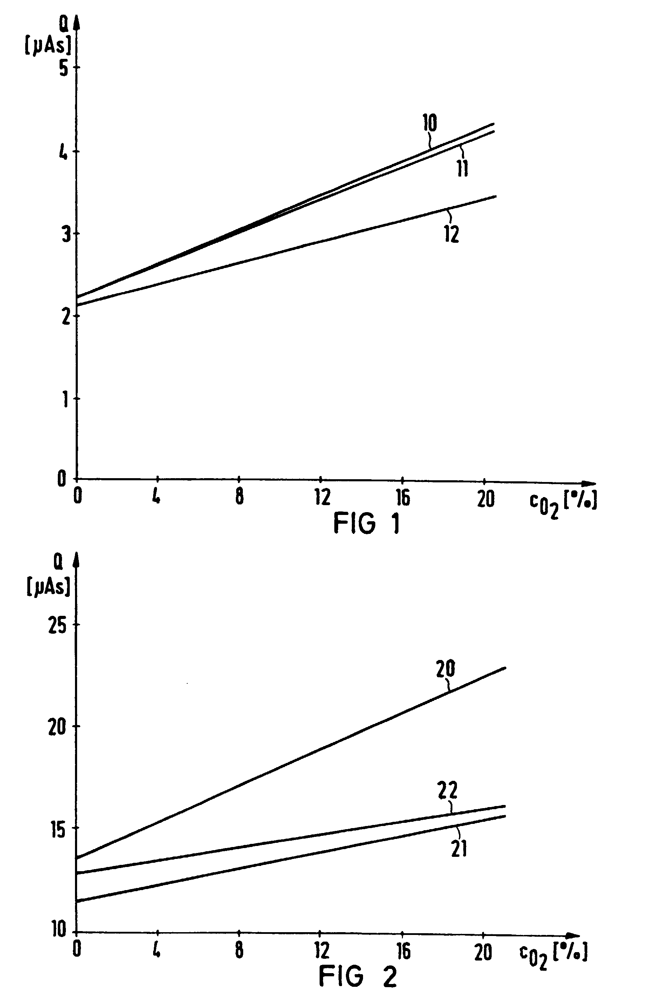

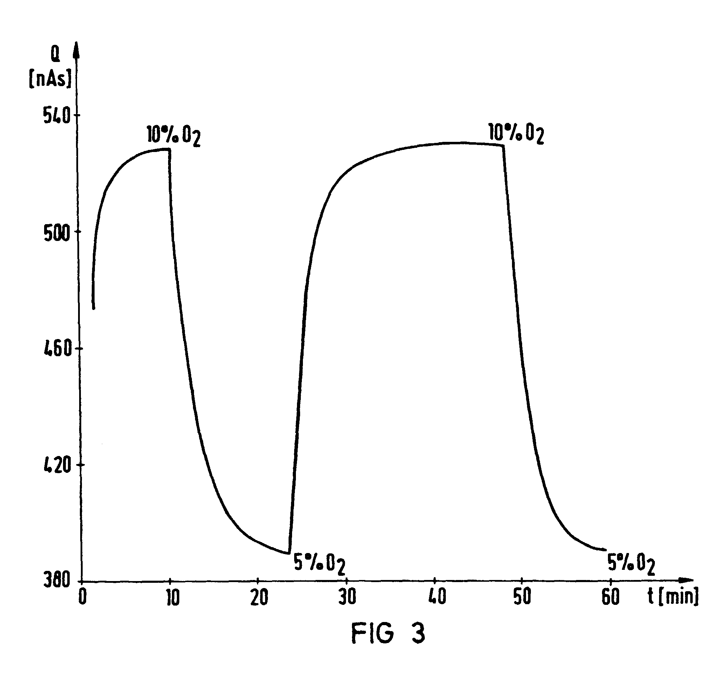

In Figur 1 und Figur 2 sind Beispiele für Eichkurven bei verschiedenen Bedingungen dargestellt (in-vitro-Messungen); dabei dient die umgesetzte Ladung Q als Maß für die Sauerstoffkonzentration c_{O₂}. Die Kurven 10 (Figur 1) und 20 (Figur 2) gelten jeweils für den Grundelektrolyten, die Kurven 11 und 21 für den Grundelektrolyten mit physiologischen Substanzen und die Kurven 12 und 22 für Rinderserum.

Bei den Eichkurven nach Figur 1 diente eine glatte Glaskohlenstoffelektrode als Meßelektrode, bei den Eichkurven nach Figur 2 eine Goldelektrode; als Gegenelektrode wurde jeweils eine Platinelektrode und als Bezugselektrode eine Ag/AgCl-Elektrode verwendet. Die Messungen wurden bei Raumtemperatur durchgeführt, d.h. bei ca. 24 bis 28°C. Bei beiden Meßelektroden betrug die Zyklendauer 1 s und die Meßperiode 50 ms. Das Erholungspotential lag bei beiden Elektroden bei ca. 0 V, das Meßpotential bei der Glaskohlenstoffelektrode bei ca. -1 V und bei der Goldelektrode bei ca. -0,8 V, jeweils bezogen auf die Ag/AgCl-Bezugselektrode.

Den Figuren 1 und 2 ist zu entnehmen, daß gute Eichkurven erhalten werden, und zwar sowohl beim Grundelektrolyten als auch bei Anwesenheit physiologischer Substanzen, d.h. von Glucose, Harnstoff und Aminosäuren, und auch bei der Verwendung von Rinderserum als Elektrolyt.

Ein beim erfindungsgemäßen Verfahren bevorzugt verwendetes Steuerungs- und Anzeigegerät, ein sogenannter O₂-Monitor, weist einen Sauerstoffsensor, d.h. eine elektrochemische Zelle mit einer Meßelektrode, einer Gegenelektrode und einer Bezugselektrode, einen Potentiostaten, einen Spannungsgeber und einen Integrator auf. Die drei Elektroden des Sauerstoffsensors sind dabei mit dem Potentiostaten verbunden. Der Spannungsgeber gibt sein Signal an den Potentiostaten, womit der Ablauf der Zyklen mit den beiden Potentialen festgelegt wird. Springt das Potential auf den negativeren Wert, d.h. auf das Meßpotential, so liefert der Spannungsgeber mit einer gewissen zeitlichen Verzögerung einen Triggerimpuls zum zeitgesteuerten Integrator. Der Integrator beginnt dann, den Strom, der über den Potentiostaten durch die Meßelektrode fließt, zu integrieren, bis nach Ablauf der Meßperiode das Potential wieder gewechselt wird; dazu ist der Integrator mit dem Potentiostaten verbunden. Das Meßsignal (in Form des Betrages der Ladung), das auf diese Weise nicht vom kapazitiven Strom der Ladung der Doppelschicht beeinflußt wird, bzw. der entsprechende Wert für die Sauerstoffkonzentration gelangt dann zur Anzeige.

Mit einem derartigen O₂-Monitor erfolgte eine Bestimmung der Sauerstoffkonzentration in Humanserum, deren Ergebnisse in Figur 3 wiedergegeben sind. Dabei wurde die Sauerstoffkonzentration stufenweise geändert, wobei zur Begasung ein Gemisch aus O₂, CO₂ und N₂ diente. Figur 3 kann entnommen werden, daß die Ansprechzeit des O₂-Sensors (als Meßelektrode diente eine Glaskohlenstoffelektrode) in Humanserum bei einer Änderung der Sauerstoffkonzentration relativ kurz ist.

## Patentansprüche

1. Anordnung zur Elektrochemischen Bestimmung der Sauerstoffkonzentration in Gewebe oder Blut, ohne die Verwendung einer Membran, umfassend
a) einen implantierbaren Sauerstoffsensor mit einer Meßelektrode bestehend aus einer Gold-, Glaskohlenstoff- oder Pyrographitelektrode und mit einer Gegenelektrode aus einem Material, das sowohl inert als auch biokompatibel ist und eine hohe Doppelschichtkapazität gegenüber der Meßelektrode aufweist,
b) Mittel zum zyklischen aufprägen der Meßelektrode von zwei Potentialen, wobei das eine Potential, das Meßpotential, im Bereich -1,4 V ≤ ϕ_{Ag/AgCl} ≤ -0,4 V und das andere Potential, das Erholungspotential, im Bereich -0,2 V ≤ ϕ_{Ag/AgCl} ≤ +0,2 V liegt; und
c) Mittel zur Auswertung des während der Meßperiode fließenden Stroms als Meßsignal,
wobei die Verweildauer beim Meßpotential zwischen 5 und 100 ms beträgt und klein ist im Vergleich zur Zyklusdauer.

2. Anordnung nach Anspruch 1, **dadurch gekennzeichnet,** daß als Gegenelektrode eine Platineelektrode verwendet wird.

3. Anordnung nach Anspruch 1 oder 2, **dadurch gekennzeichnet,** daß zusätzlich eine Bezugselektrode, insbesondere eine Ag/AgCl-Elektrode, im Sauerstoffsensor eingesetzt wird.

4. Anordnung nach einem oder mehreren der Ansprüche 1-3, **gekennzeichnet** durch einen Potentiostaten, einen Spannungsgeber und einen Integrator.

5. Verfahren zur elektrochemischen Bestimmung der Sauerstoffkonzentration in Gewebe oder Blut, ohne die Verwendung einer Membran, mittels eines Sauerstoffsensors mit einer Meßelektrode bestehend aus einer Gold-, Glaskohlenstoff- oder Pyrographitelektrode und mit einer Gegenelektrode aus einem Material, das sowohl inert als auch biokompatibel ist und eine hohe Doppelschichtkapazität gegenüber der Meßelektrode aufweist, wobei der Meßelektrode zyklisch zwei Potentiale aufgeprägt werden, wobei das eine Potential, das Meßpotential, im Bereich -1,4 V ≤ ϕ_{Ag/AgCl} ≤ -0,4 V und das andere Potential, das Erholungspotential, im Bereich -0,2 V ≤ ϕ_{Ag/AgCl} ≤ +0,2 V liegt, die Verweildauer beim Meßpotential zwischen 5 und 100 ms beträgt und klein ist im Vergleich zur Zyklusdauer und der während der Meßperiode fließende Strom als Meßsignal ausgewertet wird.

6. Verfahren nach Anspruch 5, **dadurch gekennzeichnet,** daß die Zyklusdauer zwischen 0,2 und 10 s beträgt, vorzugsweise zwischen 1 und 5 s.

7. Verfahren nach Anspruch 5 oder 6, **dadurch gekennzeichnet,** daß die Verweildauer am Meßpotential zwischen 10 und 50 ms beträgt.

8. Verfahren nach einem oder mehreren der Ansprüche 5 bis 7, **dadurch gekennzeichnet,** daß die Auswertung des Stromes mit einer zeitlichen Verzögerung in bezug auf den Beginn der Meßperiode vorgenommen wird.

9. Verfahren nach Anspruch 8, **dadurch gekennzeichnet,** daß die Auswertung des Stromes 2 bis 40 ms, vorzugsweise 10 bis 15 ms, nach Beginn der Meßperiode erfolgt.

10. Verfahren nach einem oder mehreren der Ansprüche 5 bis 8, **dadurch gekennzeichnet,** daß als Meßsignal die umgesetzte Ladung ermittelt wird.

11. Verfahren nach einem oder mehreren der Ansprüche 5 bis 10, **dadurch gekennzeichnet,** daß als Meßelektrode eine Glaskohlenstoffelktrode verwendet wird.

12. Verfahren nach einem oder mehreren der Ansprüche 5 bis 11 **dadurch gekennzeichnet,** daß als Gegenelektrode eine Platineelektrode verwendet wird.

13. Verfahren nach einem oder mehreren der Ansprüche 5 bis 12, **dadurch gekennzeichnet,** daß zusätzlich eine Bezugselektrode, insbesondere eine Ag/AgCl-Elektrode, eingesetzt wird.

## Claims

1. Arrangement for electrochemically determining the oxygen concentration in tissue or blood, without the use of a membrane, comprising
a) an implantable oxygen sensor having a measuring electrode comprising a gold electrode, a glass carbon electrode or a pyrographite electrode and having a counter electrode consisting of a material which is both inert and biocompatible and has a high double-layer capacity in comparison with the measuring electrode,
b) means for cyclically applying two potentials to the measuring electrode, the one potential, the measuring potential, lying in the range - 1.4 V ≤ ϕ_{Ag/AgCl} ≤ - 0.4 V and the other potential, the recovery potential, lying in the range - 0.2 V ≤ ϕ_{Ag/AgCl} ≤ + 0.2 V; and
c) means for evaluating the current which flows during the measuring period as a measured signal,
wherein the dwell time at the measuring potential amounts to between 5 and 100 ms and is small in comparison with the duration of the cycle.

2. Arrangement according to claim 1, characterised in that a platinum electrode is used as the counter electrode.

3. Arrangement according to claim 1 or 2, characterised in that a reference electrode, in particular an Ag/AgCl electrode, is additionally used in the oxygen sensor.

4. Arrangement according to one or more of the claims 1 to 3, characterised by a potentiostat, a voltage generator and an integrator.

5. Method for electrochemically determining the oxygen concentration in tissue or blood, without the use of a membrane, by means of an oxygen sensor having a measuring electrode comprising a gold electrode, a glass carbon electrode or a pyrographite electrode and having a counter electrode consisting of a material which is both inert and biocompatible and has a high double-layer capacity in comparison with the measuring electrode, wherein two potentials are cyclically applied to the measuring electrode, the one potential, the measuring potential, lying in the range - 1.4 V ≤ ϕ_{Ag/AgCl} ≤ - 0.4 V and the other potential, the recovery potential, lying in the range -0.2 V ≤ ϕ_{Ag/AgCl} ≤ + 0.2 V, the dwell time at the measuring potential amounts to between 5 and 100 ms and is small in comparison with the duration of the cycle and the current which flows during the measuring period is evaluated as a measured signal.

6. Method according to claim 5, characterised in that the duration of the cycle amounts to between 0.2 and 10 s, preferably between 1 and 5 s.

7. Method according to claim 5 or 6, characterised in that the dwell time at the measuring potential amounts to between 10 and 50 ms.

8. Method according to one or more of the claims 5 to 7, characterised in that the evaluation of the current is carried out with a time delay with respect to the start of the measuring period.

9. Method according to claim 8, characterised in that the evaluation of the current takes place 2 to 40 ms, preferably 10 to 15 ms, after the start of the measuring period.

10. Method according to one or more of the claims 5 to 8, characterised in that the transferred charge is established as a measured signal.

11. Method according to one or more of the claims 5 to 10, characterised in that a glass carbon electrode is used as the measuring electrode.

12. Method according to one or more of the claims 5 to 11, characterised in that a platinum electrode is used as the counter electrode.

13. Method according to one or more of the claims 5 to 12, characterised in that a reference electrode, in particular an Ag/AgCl electrode, is additionally used.

## Revendications

1. Dispositif de détermination électrochimique de la concentration en oxygène d'un tissu ou du sang, sans l'utilisation d'une membrane, comprenant
a) un détecteur implantable d'oxygène, comprenant une électrode de mesure constituée d'une électrode en or, en carbone vitreux ou en pyrographite et d'une contre-électrode en un matériau, qui est à la fois inerte et biocompatible et qui a une grande capacité de double couche vis-à-vis de l'électrode de mesure.
b) des moyens d'application cyclique à l'électrode de mesure de deux potentiels, l'un des potentiels, qui est le potentiel de mesure, étant compris entre -1,4 V ≤ ϕ_{Ag/AgCl} ≤ -0,4 V et l'autre potentiel, qui est le potentiel de régénération, étant compris entre -0,2 V ≤ ϕ_{Ag/AgCl} ≤ +0.2 V; et
c) des moyens d'exploitation, en tant que signal de mesure, du courant qui passe pendant la durée de mesure,
la durée de maintien au potentiel de mesure étant comprise entre 5 et 100 ms et étant petite par rapport à la durée du cycle.

2. Dispositif suivant la revendication 1, caractérisé en ce qu'une électrode en platine est utilisée comme contre-électrode.

3. Dispositif suivant la revendication 1 ou 2, caractérisé en ce qu'il est utilisé en plus une électrode de référence, notamment une électrode Ag/AgCl, dans le détecteur d'oxygène.

4. Dispositif suivant l'une ou plusieurs des revendications 1 à 3, caractérisé par un potentiostat, un générateur de tension et un intégrateur.

5. Procédé de détermination électrochimique de la concentration en oxygène du tissu ou du sang, sans l'utilisation d'une membrane, au moyen d'un détecteur d'oxygène ayant une électrode de mesure constituée d'une électrode en or, en carbone vitreux ou en pyrographite et d'une contre-électrode en un matériau, qui est à la fois inerte et biocompatible et qui a une grande capacité de couche double vis-à-vis de l'électrode de mesure, deux potentiels étant appliqués cycliquement à l'électrode de mesure, l'un des potentiels, le potentiel de mesure, étant compris entre -1,4 V ≤ ϕ_{Ag/AgCl} ≤ -0,4V et l'autre potentiel, qui est le potentiel de régénération, étant compris entre -0,2 V ≤ ϕ_{Ag/AgCl} ≤ + 0,2 V, la durée de maintien au potentiel de mesure étant comprise entre 5 et 100 ms et étant petite par rapport à la durée du cycle et le courant qui passe pendant la durée de mesure étant exploité en tant que signal de mesure.

6. Procédé suivant la revendication 5, caractérisé en ce que la durée du cycle est comprise entre 0,2 et 10 s, et, de préférence, entre 1 et 5 s.

7. Procédé suivant la revendication 5 ou 6, caractérisé en ce que la durée de maintien au potentiel de mesure est comprise entre 10 et 50 ms.

8. Procédé suivant l'une ou plusieurs des revendications 5 à 7, caractérisé en ce que l'exploitation du courant est effectuée avec un retard dans le temps par rapport au début de la durée de mesure.

9. Procédé suivant la revendication 8, caractérisé en ce que l'exploitation du courant s'effectue de 2 à 40 ms, de préférence de 10 à 15 ms après le début de la durée de mesure.

10. Procédé suivant l'une ou plusieurs des revendications 5 à 8, caractérisé en ce qu'il consiste à déterminer, comme signal de mesure, la charge déplacée.

11. Procédé suivant l'une ou plusieurs des revendications 5 à 10, caractérisé en ce qu'il consiste à utiliser, comme électrode de mesure, une électrode en carbone vitreux.

12. Procédé suivant l'une ou plusieurs des revendications 5 à 11, caractérisé en ce qu'il consiste à utiliser, comme contre-électrode, une électrode en platine.

13. Procédé suivant l'une ou plusieurs des revendications 5 à 12, caractérisé en ce qu'il consiste à utiliser, en plus, une électrode de référence, notamment une électrode Ag/AgCl.
